# EUROPEAN PATENT SPECIFICATION

(11) **EP 0 387 023 B1**
(45) Date of publication and mention of the grant of the patent: **18.01.1995**
(21) Application number: 90302413.1
(22) Date of filing: 07.03.1990
(51) Int. Cl.: C12P 7/64

(54) **Method for manufacturing calcium salts of fatty acids**
Verfahren zur Herstellung von Calciumsalzen von Fettsäuren
Méthode de préparation de sels calciques d'acides gras

(30) Priority: 08.03.1989 JP 53908/89
(43) Date of publication of application: 12.09.1990
(73) Proprietor: MORINAGA MILK INDUSTRY CO., LTD., Minato-ku, Tokyo-to 108 (JP); TAIYO YUSHI K.K., Yokohama City, Kanagawa Prefecture (JP)
(72) Inventor: Ishida, Shuzo, Yokohama City, Kanagawa Prefecture (JP); Ohta, Hiroaki, Hachiouji City, Tokyo (JP); Tomita, Mamoru, Yokohama-City, Kanagawa Prefecture (JP); Hayasawa, Hirotoshi, Funabashi City, Chiba Prefecture (JP)
(74) Representative: Quest, Barry

(56) References cited:
- FR-A- 2 603 900
- CHEMICAL ABSTRACTS, vol. 73, no. 1, 06 July 1970, Columbus, OH (US); J.M. CAILLAT et al., p. 26, no. 10853b#
- CHEMICAL ABSTRACTS, vol. 68, no. 10, 04 March 1968, Columbus, OH (US); G. BENZONANA, p. 4506, no. 46627s#
- CHEMICAL ABSTRACTS, vol. 108, no. 3, 18 January 1988, Columbus, OH (US); T. NISHIO et al., p. 268, no. 18320n#
- PATENT ABSTRACTS OF JAPAN, vol. 14, no. 34 (C-679)[3977], 23 January 1990#

## Description

The invention relates to a new method for manufacturing calcium salts of fatty acids, which have various and wide industrial uses.

The calcium salts of fatty acids have been used, for instance, as a stabilizer, lubricant and dispersant in the plastic industry ; as a dust removing agent in the paper industry ; as a water proofing agent in the engineering works ; and as an auxiliary agent for grinding in the ceramic industry. The calcium salt has been used also as a material for preparing cosmetic, grease, tablets and the like. Furthermore, it has been found recently that the calcium salts of fatty acids are useful for increasing the milk yield and the fat content in milk so as to be used as an additive to the feed for cows.

As for the method for manufacturing such calcium salts of fatty acids in the industrial scale, two methods have been in public knowledge, one of which is a so-called double decomposition method where an alkali metal salt which is often called alkali soap obtained by saponifying a fat and oil reacts with an aqueous calcium salt solution, while the other is a direct method where free fatty acids reacts with the same equivalent molar of calcium oxide or hydroxide.

Said methods are, however, disadvantageous in that it is necessary to heat the reactants for saponification and the reaction product for removing water therefrom, and the condensing and neutralizing reaction generates a fairly high temperature of heat, which adversely affects the product quality, above all when using the fat-and-oil predominantly containing unsaturated fatty acids. Those unsaturated fatty acids are apt to be thermally oxidized and the calcium salts obtained therefrom are deteriorated at the end of reaction to give out a peculiar bad smell. Such product can not be preferably used for the purposes referred to above, especially for the additive to the cow feed. The cows are reluctant to take such feed. It has been supposed that even if such feed is taken, such deteriorated additive would adversely affect on their physiological metabolism.

Some methods of hydrolysing fat and oil using lipase and low concentrations of calcium 2⁺ ions are known in the field of the influence of metal ions on rates of hydrolysis, namely Chemical Abstracts, Volume 73, No. 1, 1970, Abstract No. 10853b, Chemical Abstracts Vol. 68, No. 10, 1968, Abstract No. 46627s and Chemical Abstracts, Vol. 108, No. 3, 1988, Abstract No. 18320n. All of the cited documents use large amounts of water and thus would suffer from the problem of requiring water removal with its associated disadvantages as indicated above. In addition, all three prior art documents are not applied to the manufacture of calcium salts of fatty acids.

### SUMMARY OF THE INVENTION

It is, thus, an object of the invention to provide a new method for manufacturing calcium salts of fatty acids directly from the fat and oil as starting material with the least energy consumption.

It is the other object of the invention to provide the manufacturing method for the calcicum salt of unsaturated fatty acids which are apt to be readily thermally oxidized such as linoleic acid or linolenic acid without fear of the thermal deterioration.

It has been found unexpectedly that said objects can be attained simply by adding calcium hydroxide, lipase and water to liquid fat and oil and mixing with stirring to carry out uniform reaction.

### PREFERRED EMBODIMENTS OF THE INVENTION

The fat and oil to be used in the method according to the invention is selected from a class consisting of fat and oil derived from animals, plants, microorganism, those processed by partial decomposition, fractionation, hydrogenation, interesterification and so on, and mixtures thereof. It is important in the invention to be able to use the fat and oil predominantly containing the unsaturated fatty acid as the starting material.

The lipase to be used in the method of the invention is those enzymologically belonging to triacylglycerolacylhydrolase (EC 3.1.1.3). Not only lipase derived from animal pancreas but also lipases derived from plants, molds, bacteria and so on may be used.

One weight part of liquid fat and oil, which is heated at a temperature as low as possible when it is solid at the room temperature, is added with at least 0.07 weight parts, preferably 0.12-0.30 weight parts of marketed pulverized slaked lime, at least 0.01 weight parts, preferably more than 0.03 weight parts of water and at least 1,000 Units (hereinafter referred to as U), preferably 3,000 U - 15,000 U of lipase in relation to 100g of the fat and oil and homogeneously mixed with stirring to carry our uniform reaction.

Addition of water, slaked lime and lipase to the fat and oil may be made in the optional order. It is preferable, however, to separately prepare a solution or dispersion of lipase in water, which is then added. The reaction time is considerably varied depending on the sort of the used fat and oil, titer and amount of used lipase and amount of water added.

After the lapse of stirring for a desired length of time, the reaction mixture is taken out and kept still at the room temperature, which is then crushed to be in desired size for the finished product.

The amount of lipase to be added is very small as seen from the above and the following Examples as well as Experiments so that the product can be used as it is for various purposes. It is possible, however, to remove still remaining lipase and produced glycerol during the reaction by water washing if necessary. The amount of water for washing is varied depending on the purpose of using the calcium salts of fatty acids. When it is used as the additive to the feed for cows, the amount of water to be used is preferably as small as possible in order not to necessitate an additional aqueous phase removal from the reaction product. The amount of slaked lime to be used is preferably as small as possible lest excessive amount of calcium should remain in the reaction product so far as the reaction may be carried out without hindrance.

The calcium salts of fatty acids obtained according to the invention may be used just like as the calcium salts obtained according to the prior art. For instance when calcium stearate obtained from hydrogenated beef tallow is combined in pencil black lead comprising crystaline graphite in the amount of about 10 %, sliding of the formed lead in the extruder die and writing property are improved. When such calcium salt is added in a fire-proof board for building which mainly comprises calcium silicate, in the amount of 5-10 %, the water-proofness thereof is fairly improved.

The invention will be explained in more detail in the following Experiments and Examples.

### Experiment 1

As to the effect of the amounts of water and lipase on forming of the calcium salt of fatty acid, the following experiment was carried out.

### (1) Forming of Calcium Salts of Fatty Acids

Marketed soyabean oil (manufactured by Taiyo Yushi K.K.) in the amount of 1 kg was added with 0.14 kg of marketed slaked lime (manufactured by Kanto Kagaku K.K.), marketed lipase derived from microorganism (30U/mg, manufactured by Amano Seiyaku K.K.) and water of which amounts are shown in Table 1 to be given hereafter and subjected to treatments as shown in following Example 2 so as to manufacture the corresponding calcium salts of fatty acids.

### (2) Experiment of Reaction Products

Each one sample extracted from the reaction products was dissolved in chloroform and subjected to thin layer chromatography so as to confirm if any unreacted triglyceride (soybean oil) remains in the reaction products and observe water separation.

### (3) Results are shown in Table 1.

**Table 1**

| Effects of Amounts of Used Water and Lipase on Forming of Calcium Salts of Fatty Acids | | | | | | |
|---|---|---|---|---|---|---|
| Water (kg) | Calcium Salts Formed Depending on Lipase Amount (U) | | | | | |
| | 5 × 10³ | 1 × 10⁴ | 3 × 10⁴ | 5 × 10⁴ | 1 × 10⁵ | 3 × 10⁵ |
| 0.005 | X | X | X | X | X | X |
| 0.01 | X | X | ○ | ○ | ○ | ○ |
| 0.03 | X | ○ | ○ | ○ | ○ | ○ |
| 0.10 | X | ○ | ○ | ○ | ○ | ○ |
| 0.30 | X | ○ | ○ | ○ | ○ | ○ |
| 0.50 | X | △ | △ | △ | △ | △ |

Symbols used in Table 1 mean followings.
- ○: Hydrolysis was complete so as to form calcium salts of fatty acids.
- △: Hydrolysis was complete so as to form calcium salts of fatty acids, but some water separation was observed.
- X: Hydrolysis was incomplete so that some triglyceride (soybean oil) was observed.

### (4) Valuation

Considering the results shown in Table 1, it can be concluded that if 1 kg of soybean oil is added with lipase in the amount more than 1.0 x 10⁴ U and water in the amount of 0.01-0.3 kg the fatty acid calcium salt may be formed satisfactorily without causing separation of aqueous phase, that if water is added in the amount more than 0.5 kg the reaction is completely finished but aqueous phase is separated, and that if water is added in the amount less than 0.005 kg the yield of the calcium salt of fatty acid is lowered.

If the amount of lipase to be added to 1 kg of the fat and oil is more than 1.0 x 10⁴ U and if the amount of water to be added thereto is more than 0.03 kg, the fat and oil may be completely decomposed and all fatty acid may be converted to the calcium salt.

Similar results have been obtained from the experiments carried out with changing the sort of the fat and oil and the amount of slaked lime.

### Experiment 2

As to the effect of the amounts of slaked lime on forming of the calcium salt of fatty acid , the following experiment was carried out.

### (1) Forming of Calcium Salts of Fatty Acids.

Marketed rapeseed oil (manufactured by Taiyo Yushi K.K.) in the amount of 1kg was added with marketed slaked lime powder (manufactured by Kanto Kagaku K.K.) in the amounts to be shown in Table 2, marketed lipase derived from microorganism (30 U/mg, manufactured by Amano Seiyaku K.K.) in the amount of 1g and 0.05kg of water and subjected to treatments as shown in Example 2 so as to manufacture the corresponding fatty acid calcium salts of fatty acids.

### (2) Experiment of Reaction Products

Each one sample drawn out of the uniformly mixed reaction products was extracted with a mixture of n-hexane/ether (1:1:v/v) so as to determine the amounts of unreacted glyceride according to the conventional weight method.

### (3) Results are shown in Table 2,

**Table 2**

| Effects of Amounts of Used Slaked Lime on Forming of Calcium Salts of Fatty Acids | |
|---|---|
| Slaked Lime (kg) | Unreacted Glyceride Amount (weight % ) |
| 0.05 | 50 |
| 0.07 | 35 |
| 0.12 | 0 |
| 0.30 | 0 |
| 0.50 | 0 |

### (4) Valuation

It can be concluded from the above that if slaked lime is used in the amount more than 0.12 weight parts in relation to 1 part of the fat and oil, rapeseed oil glyceride may be completely reacted so that the calcium salt of fatty acid may be satisfactorily formed, that if the amount of slaked lime to be added to the 1 weight part of fat and oil is more than 0.50 weight parts, the reaction may proceed with but slaked lime remains unreacted in the amount more than 0.30 weight parts so that such reaction product is not preferable e.g. as
the additive to the feed, and that if slaked lime is used in the amount less than 0.07 parts, unreacted glyceride remains in the amount more than 35 % relative to the product so as to exude out thereof.

Similar results have been obtained from the experiments carried out with changing the sorts of fat and oil and lipase.

### Example 1

Marketed soybean oil (manufactured by Taiyo Yushi K.K.) in the amount of 10 kg was uniformly mixed with 1.4 kg of marketed slaked lime (manufactured by Kanto Kagaku K.K.) to which 7g of marketed lipase derived from pig pancreas (70U/mg, produced by Sigma) in 1000 ml of water was added and mixed with stirring at the room temperature for about 30 minutes and then kept still for about 6 hours in order to complete the reaction. The reaction product containing about 10 kg of the corresponding fatty acid calcium salt of fatty acid was obtained in the amount of 12.4 kg.

### Example 2

Marketed corn oil (manufactured by Taiyo Yushi K.K.) in the amount of 20 kg was uniformly mixed with 2.8 kg of marketed slaked lime (manufactured by Kanto Kagaku K.K.), to which 40g of lipase derived from microorganism (30 U/mg, manufactured by Amano Seiyaku K.K.) in 200 ml of water was mixed with stirring at the room temperature for about 20 minutes and then kept still for 8 hours in order to complete the reaction. Thereby the solid reaction product containing about 20 kg of the corresponding calcium salt of fatty acid was obtained in the amount of 23 kg, which was then crushed to be in the finished product in the amount of about 22.5 kg.

### Example 3

Marketed beef tallow (manufactured by Taiyo Yushi K.K.) in the amount of 10 kg was heated at 40°C to be melted and uniformly mixed with 18g of marketed lipase derived from microorganism (500U/mg manufactured by Sigma) and 500 mℓ of water, to which 1.5 kg of marketed slaked lime (manufactured by Kanto Kagaku K.K.) was uniformly mixed with stirring for about 30 minutes and then kept still for about 7 hours to be reacted so that the reaction product containing about 10kg of the corresponding fatty acid calcium salt in the amount of 12 kg, which was washed with water, dried by mildly heating, and crushed to be in about 9 kg of finished product.

Since the reaction can be carried out under mild conditions as referred to above, the reaction product may be obtained in improved quality. Furthermore, the product can be obtained directly from the fat and oil so that the manufacturing cost therefore may be considerably saved.

## Claims

1. A method for manufacturing calcium salts of fatty acids, which comprises reacting one part by weight of a fat and oil with at least 0.07 parts by weight of calcium hydroxide and at least 0.01 parts by weight of water in the presence of at least 10 units of triacylglycerolacylhydrolase (EC 3.1.1.3) per gram of the fat and oil by agitating.

2. The method as claimed in claim 1, wherein the solid fat and oil at the room temperature is heated at the temperature as low as possible to obtain a uniform mixture.

## Patentansprüche

1. Verfahren zur Herstellung von Kalziumsalzen aus Fettsäure, das die Reaktion eines Gewichtsteils Fett und Öl mit mindestens 0,07 Gewichtsteilen Kalziumhydroxid und mindestens 0,01 Gewichtsteil Wasser bei Vorhandensein von mindestens 10 Einheiten Triacrylglyzerinacrylhydrolase (EC 3.1.1.3) pro Gramm Fett und Öl durch Rühren zum Inhalt hat.

2. Verfahren nach Anspruch 1, bei dem das bei Zimmertemperatur feste Fett und Öl auf die geringste erforderliche Temperatur erhitzt wird, um ein gleichmäßiges Gemisch zu erhalten.

## Revendications

1. Procédé pour la fabrication de sels de calcium d'acides gras, qui consiste à faire réagir 1 partie en poids d'une graisse ou huile avec au moins 0,07 partie en poids d'hydroxyde de calcium et au moins 0,01 partie en poids d'eau en présence d'au moins 10 unités de triacylglycérolacylhydrolase (EC 3.1.1.3) par gramme de la graisse ou huile sous agitation.

2. Procédé tel que revendiqué dans la revendication 1, dans lequel la graisse ou huile solide à la température ambiante est chauffée à une température aussi basse que possible pour donner un mélange uniforme.
